# EUROPEAN PATENT APPLICATION

(11) **EP 3 967 224 A1**
(43) Date of publication of application: **16.03.2022**
(21) Application number: 20810402.6
(22) Date of filing: 29.01.2020
(51) Int. Cl.: A61B 5/11, A61B 5/16, G10L 15/28, G06F 3/01, G06F 3/16

(54) **INFORMATION PROCESSING METHOD, INFORMATION PROCESSING SYSTEM, AND INFORMATION PROCESSING PROGRAM**

(30) Priority: 17.05.2019 JP 2019093364
(71) Applicant: Panasonic Intellectual Property Management Co., Ltd., Osaka-shi, Osaka 540-6207 (JP)
(72) Inventor: HARADA, Masaaki, Osaka-shi, Osaka (JP)
(74) Representative: Grünecker Patent- und Rechtsanwälte PartG mbB
(86) International application number: PCT/JP2020/003261
(87) International publication number: WO 2020/235141

(57) **Abstract**

A server (2) acquires biological data from a biosensor that senses a biological state of a user; acquires sound data from a microphone that senses sound in space in which the user is present; determines a sleep state of the user based on the biological data; converts the sound data into text data; and controls output of a device control command based on the text data by controlling necessity of operation determination to determine whether a predetermined operation has been performed by the user based on the sleep state.

## Description

### Technical Field

The present disclosure relates to a technique for controlling a device by a user's voice.

### Background Art

Conventionally, a device control system that controls a device by a user's voice is known. The conventional device control system includes, for example, a smart speaker, a server, and a device. To begin with, the smart speaker receives voice input by the user to operate the device. Then, the server recognizes the input voice and transmits a control command corresponding to the user's operation to the device. The device operates according to the received control command.

In such a device control system, in order to prevent erroneous operations of the device due to unintended words spoken by the user, the user needs to first speak a wake word when operating the device.

For example, Patent Literature 1 discloses a wearable terminal device that captures images with a digital camera and a line-of-sight recognition camera, detects the line of sight from image data captured by the line-of-sight recognition camera, performs image recognition on an area corresponding to the detected line of sight in the image captured by the digital camera, and controls display of a monitor display unit in a set operation mode among a plurality of operation modes when a barcode is recognized by the image recognition.

However, if the above-described conventional technique is used during a period from lying in bed to rising (hereinafter also referred to as during sleeping period), there is a possibility of increasing the user's awakening degree.

### Citation List

### Patent Literature

Patent Literature 1: Japanese Patent Application Laid-Open No. 2011-14082

### Summary of Invention

The present disclosure has been made to solve the above-described problem. An object of the present disclosure is to provide a technique that can suppress the increase in user's awakening degree while maintaining the convenience of device control during the sleeping period.

An information processing method according to one aspect of the present disclosure includes, by a computer: acquiring biological data from a biosensor that senses a biological state of a user; acquiring sound data from a microphone that senses sound in space in which the user is present; determining a sleep state of the user based on the biological data; converting the sound data into text data; and controlling output of a device control command based on the text data by controlling necessity of operation determination to determine whether a predetermined operation has been performed by the user based on the sleep state.

The present disclosure can suppress the increase in user's awakening degree while maintaining the convenience of device control during the sleeping period.

### Brief Description of Drawings

FIG. 1 is a diagram showing one example of a configuration of a voice control system in a first embodiment of the present disclosure.
FIG. 2 is a diagram showing a configuration of a server in the first embodiment of the present disclosure.
FIG. 3 is a diagram showing one example of a command table stored in a command table storage unit in the first embodiment of the present disclosure.
FIG. 4 is a flowchart showing one example of an information processing method by the server in the first embodiment of the present disclosure.
FIG. 5 is a diagram showing one example of a configuration of a voice control system in a second embodiment of the present disclosure.
FIG. 6 is a diagram showing a configuration of a server in the second embodiment of the present disclosure.

### Description of Embodiments

### (Underlying Knowledge Forming Basis of Present Disclosure)

In a conventional device control system, a user needs to speak a wake word before controlling a device by voice. The device control system continuously acquires the voice spoken by the user. When the wake word is included in the acquired voice, the device control system transmits a control command corresponding to the voice following the wake word to the device.

Therefore, causing a user who lies in bed or a user who is awake halfway to speak the wake word before speaking a voice for controlling the device will cause the user to perform unnecessary operations, and there is a possibility of impairing the user's convenience. There is a possibility of increasing the awakening degree of the user who is going to sleep or the user who is not completely awake. Processing is required to detect the wake word, and there is a possibility that the processing load of the device control system will increase.

As described above, in the conventional technique, the user needs to wear a head mount display for detecting the line of sight. Therefore, for the user who operates the device when going to sleep, there is a possibility that the worn head mount display may be a factor of hindering comfortable sleep.

In order to solve the above problem, an information processing method according to one aspect of the present disclosure includes, by a computer: acquiring biological data from a biosensor that senses a biological state of a user; acquiring sound data from a microphone that senses sound in space in which the user is present; determining a sleep state of the user based on the biological data; converting the sound data into text data; and controlling output of a device control command based on the text data by controlling necessity of operation determination to determine whether a predetermined operation has been performed by the user based on the sleep state.

This configuration allows the user, for example, to control the device by voice without predetermined operations such as speaking the wake word, making it possible to suppress the increase in the user's awakening degree while maintaining the convenience of device control during the sleeping period. The necessity of predetermined operation is controlled according to the sleep state of the user, making it possible to suppress erroneous control of the device without predetermined operations, which leads to an increase in the convenience of controlling the device by the user's voice.

In the information processing method described above, the control of the output of the device control command may include: determining the necessity of the operation determination based on the sleep state; when it is determined that the operation determination is necessary, determining whether the predetermined operation has been performed by the user, and outputting the device control command when it is determined that the predetermined operation has been performed; and when it is determined that the operation determination is unnecessary, outputting the device control command.

With this configuration, for example, when the user lies in bed or when the user awakes halfway, the device control command is output without operation determination. Therefore, it is possible to further enhance the convenience of controlling the device by the user's voice during the sleeping period.

The information processing method described above may further include acquiring environmental information indicating an environmental state in the space, and the control of the output of the device control command may include determining the necessity of the operation determination based on the sleep state and the environmental information.

With this configuration, it is possible to determine the necessity of operation determination by considering not only the sleep state but also whether the environmental state in the space is suitable for sleeping.

In the information processing method described above, the control of the output of the device control command may include transmitting the device control command to a device associated with the device control command.

With this configuration, the device control command is transmitted to the device associated with the device control command, and thus the device control by voice can be implemented.

In the information processing method described above, the determination of the necessity of the operation determination may include: determining whether the sleep state is a lying in bed state in which the user lies in bed; and determining that the operation determination is unnecessary when it is determined that the sleep state is the lying in bed state.

With this configuration, when the user's sleep state is the lying in bed state in which the user has lain in bed, the device can be controlled without operation determination. Therefore, it is possible to further enhance the convenience of controlling the device by the user's voice between the time of lying in bed and the time of falling asleep.

In the information processing method described above, the determination of the necessity of the operation determination may include: determining whether the sleep state is a nocturnal awakening state in which the user awakes during a sleeping period from lying in bed to rising; and determining that the operation determination is unnecessary when it is determined that the sleep state is the nocturnal awakening state.

With this configuration, when the user's sleep state is the nocturnal awakening state in which the user awakes during the sleeping period from lying in bed to rising, the device can be controlled without operation determination. Therefore, it is possible to further enhance the convenience of controlling the device by the user's voice in the nocturnal awakening state. In addition, it is possible to suppress the awakening degree from increasing due to the device operation.

In the information processing method described above, the determination of the necessity of the operation determination may include: determining whether the sleep state is a nocturnal awakening sign state indicating a sign of shifting to the nocturnal awakening state in which the user awakes during the sleeping period from lying in bed to rising; and determining that the operation determination is unnecessary when it is determined that the sleep state is the nocturnal awakening sign state.

With this configuration, when the user's sleep state is the nocturnal awakening sign state indicating the sign of shifting to the nocturnal awakening state in which the user awakes during the sleeping period from lying in bed to rising, the device can be controlled without operation determination. Therefore, it is possible to further enhance the convenience of controlling the device by the user's voice in the nocturnal awakening sign state. In addition, it is possible to enhance responsiveness to the user's voice when shifting to the nocturnal awakening state.

In the information processing method described above, the determination of the necessity of the operation determination may include: determining whether the sleep state is a first awake state from when the user lies in bed to falling asleep; and determining that the operation determination is unnecessary when it is determined that the sleep state is the first awake state.

With this configuration, when the user's sleep state is the first awake state from when the user lies in bed to falling asleep, the device can be controlled without operation determination. Therefore, it is possible to further enhance the convenience of controlling the device by the user's voice in the first awake state.

In the information processing method described above, the determination of the necessity of the operation determination may include: determining whether the sleep state is a second awake state during a period from a scheduled rising time of the user to a predetermined time; and determining that the operation determination is unnecessary when it is determined that the sleep state is the second awake state.

With this configuration, when the user's sleep state is the second awake state during the period from the scheduled rising time to the predetermined time, the device can be controlled without operation determination. Therefore, it is possible to further enhance the convenience of controlling the device by the user's voice in the second awake state.

In the information processing method described above, the determination of the necessity of the operation determination may include: determining whether the sleep state is a state from when the user lies in bed to rising; and determining that the operation determination is unnecessary when it is determined that the sleep state is the state from when the user lies in bed to rising.

Since this configuration allows the device to be controlled without predetermined operation even in a stage of sleep preparation, it is possible to suppress the awakening degree of a person who is about to sleep from increasing. Even in the stage of preparation for rising from bed, it is possible to suppress the person's awakening degree from increasing before rising from bed completely.

In the information processing method described above, the control of the output of the device control command may include generating the device control command based on the predetermined sleep state when it is determined that the sleep state is the predetermined sleep state.

With this configuration, when it is determined that the sleep state is the predetermined sleep state, the device control command is generated based on the predetermined sleep state. Therefore, the device can be controlled according to the current sleep state of the user, and a comfortable sleeping environment for the user can be implemented.

In the information processing method described above, the generation of the device control command may include generating the device control command according to a time length from the scheduled rising time of the user to current time when it is determined that the sleep state is the predetermined sleep state.

With this configuration, for example, the device can be controlled differently between when the user awakes immediately before rising and when the user awakes before predetermined time of the scheduled rising time, and the convenience of controlling the device by the user's voice can be further improved.

In the information processing method described above, the control of the output of the device control command may include generating only the predetermined device control command related to going to sleep of the user when it is determined that the sleep state is the predetermined sleep state.

With this configuration, when it is determined that the sleep state is the predetermined sleep state, only the predetermined device control command related to the user's going to sleep is generated. Therefore, when the device is controlled without operation determination, it is possible to prevent erroneous operation of the device due to an unintended word spoken by the user.

In the information processing method described above, the predetermined operation may include any of speaking a predetermined wake word by the user, a predetermined gesture by the user, and predetermined line-of-sight movement by the user.

This configuration makes it possible to control the device by voice based on the sleep state of the user without determining speaking the predetermined wake word by the user, the predetermined gesture by the user, and the predetermined line-of-sight movement by the user. Therefore, it is possible to further enhance the convenience of controlling the device by the user's voice when going to sleep.

An information processing system according to another aspect of the present disclosure includes: a biosensor configured to sense a biological state of a user; a microphone configured to sense a sound in space in which the user is present; and an information processing device, wherein the information processing device includes: a biological data acquisition unit configured to acquire biological data from the biosensor; a sound data acquisition unit configured to acquire sound data from the microphone; a sleep state determination unit configured to determine a sleep state of the user based on the biological data; a conversion unit configured to convert the sound data into text data; an output control unit configured to control output of a device control command based on the text data by controlling necessity of operation determination to determine whether a predetermined operation has been performed by the user based on the sleep state.

This configuration allows the user, for example, to control the device by voice without predetermined operations such as speaking the wake word, making it possible to suppress the increase in the user's awakening degree while maintaining the convenience of device control during the sleeping period. The necessity of predetermined operation is controlled according to the sleep state of the user, making it possible to suppress erroneous control of the device without predetermined operations, which leads to an increase in the convenience of controlling the device by the user's voice.

An information processing program according to another aspect of the present disclosure causes a computer to perform functions of: acquiring biological data from a biosensor that senses a biological state of a user; acquiring sound data from a microphone that senses sound in space in which the user is present; determining a sleep state of the user based on the biological data; converting the sound data into text data; and controlling output of a device control command based on the text data by controlling necessity of operation determination to determine whether a predetermined operation has been performed by the user based on the sleep state.

This configuration allows the user, for example, to control the device by voice without predetermined operations such as speaking the wake word, making it possible to suppress the increase in the user's awakening degree while maintaining the convenience of device control during the sleeping period. The necessity of predetermined operation is controlled according to the sleep state of the user, making it possible to suppress erroneous control of the device without predetermined operations, which leads to an increase in the convenience of controlling the device by the user's voice.

Embodiments of the present disclosure will be described below with reference to the drawings. Note that the following embodiments are one example embodying the present disclosure, and do not limit the technical scope of the present disclosure.

### (First Embodiment)

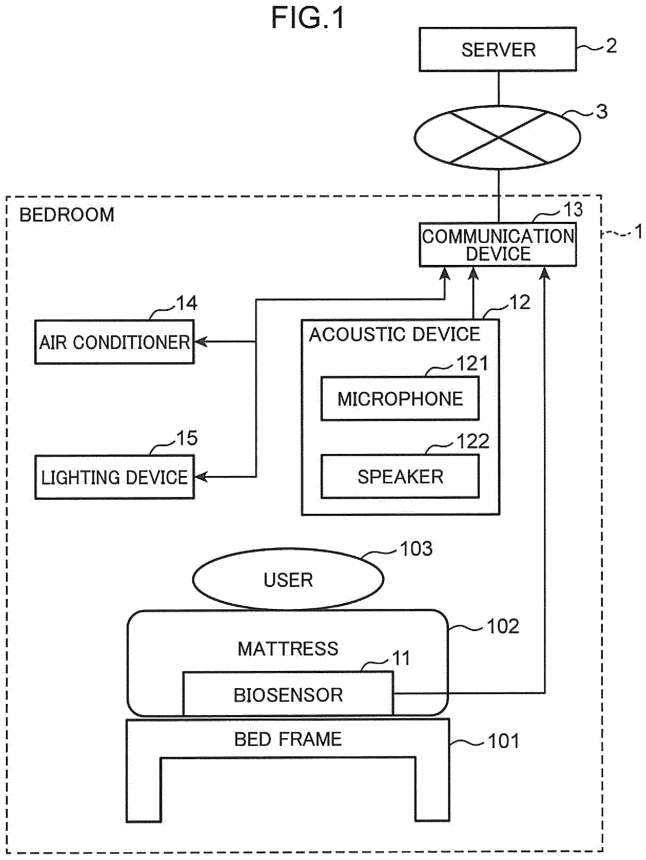

FIG. 1 is a diagram showing one example of a configuration of a voice control system in a first embodiment of the present disclosure.

In FIG. 1, the voice control system includes a server 2, a biosensor 11, an acoustic device 12, a communication device 13, an air conditioner 14, and a lighting device 15. The biosensor 11, the acoustic device 12, the communication device 13, the air conditioner 14, and the lighting device 15 are disposed in a bedroom 1.

The biosensor 11 is, for example, a Doppler sensor that senses a biological state of a user 103. The biosensor 11 is installed between a mattress 102 and a bed frame 101 to sense the biological state of the user 103. The biosensor 11 detects a biological state from body movement of the user 103 who is sleeping. The biological state is, for example, the heart rate, respiratory rate, and body movement amount. The biosensor 11 transmits biological data indicating the biological state to the communication device 13.

Note that in the first embodiment, the biosensor 11 is installed between the mattress 102 and the bed frame 101, but the present disclosure is not particularly limited to this example. The biosensor 11 may be disposed on the ceiling of the bedroom 1 above the mattress 102. The biosensor 11 may not be a Doppler sensor but may be another sensor capable of measuring the biological data.

The acoustic device 12 includes a microphone 121 and a speaker 122. Note that in the first embodiment, the acoustic device 12 does not have to include the speaker 122.

The microphone 121 senses sound in space in which the user is present. The space in which the user is present is the bedroom 1. The microphone 121 collects the voice spoken by the user 103 and transmits voice data to the communication device 13.

The speaker 122 outputs the voice to the outside.

The air conditioner 14 is disposed in the bedroom 1 in which the user 103 goes to sleep. The air conditioner 14 performs a cooling operation, a heating operation, or a dehumidifying operation, and adjusts the temperature and/or humidity in the bedroom 1. The air conditioner 14 receives a device control command transmitted by the server 2 and controls the operation based on the received device control command. Note that the air conditioner 14 may control the operation based on a device control command from a remote controller.

The lighting device 15 is disposed in the bedroom 1 in which the user 103 goes to sleep. The lighting device 15 adjusts brightness in the bedroom 1. The lighting device 15 receives a device control command transmitted by the server 2 and controls the brightness based on the received device control command. Note that the lighting device 15 may control the brightness based on a device control command from a remote controller. The lighting device 15 includes a lighting device disposed on the ceiling of the bedroom 1 and a lighting device disposed at the feet of the user 103.

The communication device 13 is, for example, a router, and is communicably connected to the biosensor 11, the acoustic device 12, the air conditioner 14, and the lighting device 15 via a local area network (LAN). The communication device 13 is communicably connected to the server 2 via a network 3. The biosensor 11, the acoustic device 12, the air conditioner 14, and the lighting device 15 communicate with the server 2 via the communication device 13.

The communication device 13 transmits the biological data detected by the biosensor 11 to the server 2. The communication device 13 transmits the voice data collected by the microphone 121 to the server 2. The communication device 13 receives the device control command for controlling the air conditioner 14 transmitted by the server 2, and transmits the received device control command to the air conditioner 14. The communication device 13 receives the device control command for controlling the lighting device 15 transmitted by the server 2, and transmits the received device control command to the lighting device 15.

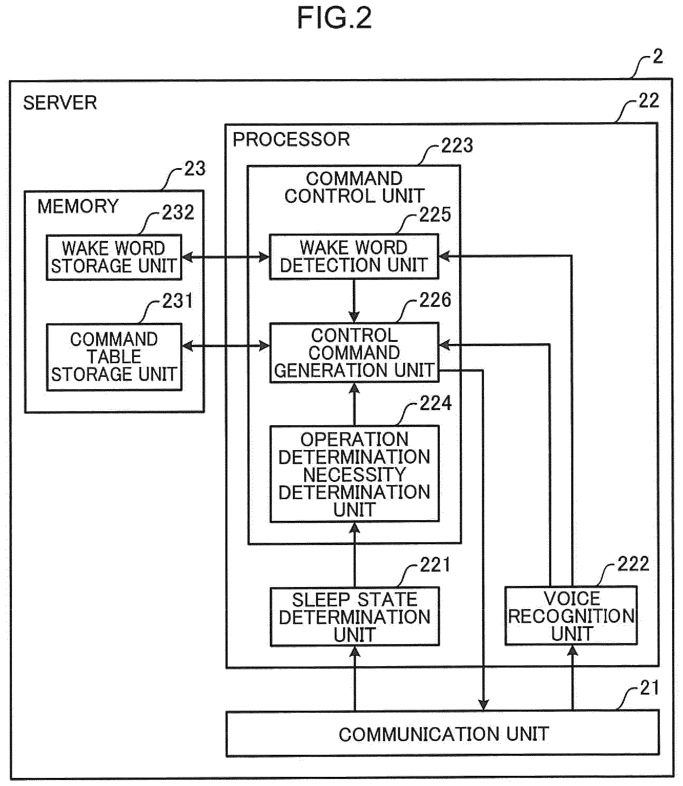

FIG. 2 is a diagram showing a configuration of the server in the first embodiment of the present disclosure.

The server 2 shown in FIG. 2 includes a communication unit 21, a processor 22, and a memory 23.

The communication unit 21 acquires the biological data from the biosensor 11. The communication unit 21 receives the biological data transmitted by the biosensor 11. The communication unit 21 acquires sound data from the microphone 121. The communication unit 21 receives the sound data transmitted by the acoustic device 12.

The processor 22 includes a sleep state determination unit 221 (first determination unit), a voice recognition unit 222, and a command control unit 223.

The memory 23 is, for example, a non-volatile semiconductor memory, and includes a command table storage unit 231 and a wake word storage unit 232.

The sleep state determination unit 221 determines the sleep state of the user 103 based on the biological data. The sleep state determination unit 221 determines the sleep state of the user 103 at predetermined time intervals from the biological data received by the communication unit 21. The predetermined time is, for example, 1 minute. Examples of the sleep state include a leaving bed state in which the user 103 is not on bedding, a lying in bed state in which the user 103 has lain in bed, an awake state in which the user 103 is awake on the bedding, a pre-sleep awake state (first awake state) in which the user 103 is awake during a period from lying in bed to falling asleep or is awake continuously for a predetermined period after lying in bed, a REM sleep state, a light sleep state in which the user 103 is in non-REM sleep of stage 1 or stage 2, a deep sleep state in which the user 103 is in non-REM sleep of stage 3 or stage 4, a nocturnal awakening state in which the user 103 awakes during the sleep period from lying in bed to rising, a nocturnal awakening sign state indicating a sign that the user 103 shifts to the nocturnal awakening state, a halfway leaving bed state in which the user 103 leaves the bed during the sleeping period, and a pre-rising awake state (second awake state) in which the user 103 awakes within a period from scheduled rising time to a predetermined time.

Here, a depth level of sleep of a person will be described.

Sleep of a person can be classified into a plurality of sleep states that changes on a time-series basis according to the depth of sleep or the characteristics of sleep. Sleep is classified into REM sleep and non-REM sleep. REM sleep is sleep that involves high-speed eyeball movement. In REM sleep, the body is in a resting state, but the brain is in an active state. It is said that a person often has a dream during REM sleep. Non-REM sleep is sleep that does not involve high-speed eyeball movement, and is further classified into four stages from stage 1 to stage 4 according to the depth of sleep. Stage 4 is the deepest sleep level. Usually, sleep reaches stage 3 or stage 4 of non-REM sleep within 45 to 60 minutes after falling asleep, and then gradually becomes lighter and becomes REM sleep in about 1 to 2 hours. After that, it is said that non-REM sleep and REM sleep are repeated alternately in a sleep cycle of 90 to 110 minutes, but this also has large variations among individuals or variations in one individual.

The biological data of the body movement amount, respiratory rate, and heart rate are correlated with the sleep state. For example, in a deep sleep state such as stage 3 or stage 4 of non-REM sleep, it is known that the body movement amount is small and the heart rate variability (RRI) is low. The sleep state determination unit 221 uses such a correlation to determine the sleep state of a person from the biological data in real time. Based on the biological data, the sleep state determination unit 221 estimates which of the leaving bed state, lying in bed state, awake state, pre-sleep awake state, REM sleep state, light sleep state, deep sleep state, nocturnal awakening state, nocturnal awakening sign state, halfway leaving bed state, and pre-rising awake state is the sleep state of the user 103.

Note that when the biological data of the user 103 is not detected, the sleep state determination unit 221 determines that the sleep state is the leaving bed state. When the biological data is detected from the state in which the biological data of the user 103 is not detected, the sleep state determination unit 221 determines that the sleep state is the lying in bed state. When the sleep state is an awake state after it is determined that the sleep state is the lying in bed state until falling asleep, the sleep state determination unit 221 determines that the sleep state is the pre-sleep awake state. When the sleep state is an awake state continuously for a predetermined period after it is determined that the sleep state is the lying in bed state, the sleep state determination unit 221 determines that the sleep state is the pre-sleep awake state. Note that the predetermined period is, for example, 15 minutes.

In a case where the body movement amount increases above a threshold or the heart rate increases above a threshold when the sleep state is the REM sleep state, the light sleep state, or the deep sleep state, the sleep state determination unit 221 determines that the sleep state is the nocturnal awakening sign state. Note that the heart rate or the respiratory rate increases as approaching the awake state. Therefore, the sleep state determination unit 221 may determine that the sleep state is the nocturnal awakening sign state in a case where a moving average of the heart rate or the respiratory rate increases above a threshold when the sleep state is the REM sleep state, the light sleep state, or the deep sleep state.

When the sleep state changes from the REM sleep state, the light sleep state, or the deep sleep state to the awake state, and when the time of change to the awake state is before the scheduled rising time by a predetermined time or more, the sleep state determination unit 221 determines that the sleep state is the nocturnal awakening state. Note that the scheduled rising time is input in advance by the user 103 and stored in the memory 23. The predetermined time is, for example, 15 minute.

After the sleep state changes from the REM sleep state, the light sleep state, or the deep sleep state to the awake state, when the user 103 leaves the bed and the time when the user leaves the bed is before the scheduled rising time by a predetermined time or more, the sleep state determination unit 221 determines that the sleep state is the halfway leaving bed state. Note that the predetermined time is, for example, 15 minutes.

When the sleep state changes from the REM sleep state, the light sleep state, or the deep sleep state to the awake state, and when the time from the time of change to the awake state to the scheduled rising time is within a predetermined time, the sleep state determination unit 221 determines that the sleep state is the pre-rising awake state. Note that the predetermined time is, for example, 15 minutes.

Note that in the first embodiment, non-REM sleep of stage 1 or stage 2 corresponds to the light sleep state, and non-REM sleep of stage 3 or stage 4 corresponds to the deep sleep state, but the present disclosure is not particularly limited to this example. Non-REM sleep of stage 1 may correspond to the light sleep state, and non-REM sleep of stage 2, stage 3, or stage 4 may correspond to the deep sleep state. Non-REM sleep of stage 1, stage 2, or stage 3 may correspond to the light sleep state, and non-REM sleep of stage 4 may correspond to the deep sleep state.

In the first embodiment, the sleep state determination unit 221 distinguishes between the REM sleep state, the light sleep state, and the deep sleep state, but the present disclosure is not particularly limited to this example. Without distinguishing between the REM sleep state, the light sleep state, and the deep sleep state, the sleep state determination unit 221 may determine these states as one sleep state.

The voice recognition unit 222 converts sound data into text data. The voice recognition unit 222 receives digitized sound data from the communication unit 21 and converts the sound data into text data by using an acoustic model or a language model. The acoustic model or the language model is stored in the memory 23.

The command table storage unit 231 stores a command table that associates a control instruction word indicating a specific language spoken to instruct device control with a control target device, and the device control command indicating control contents of the device.

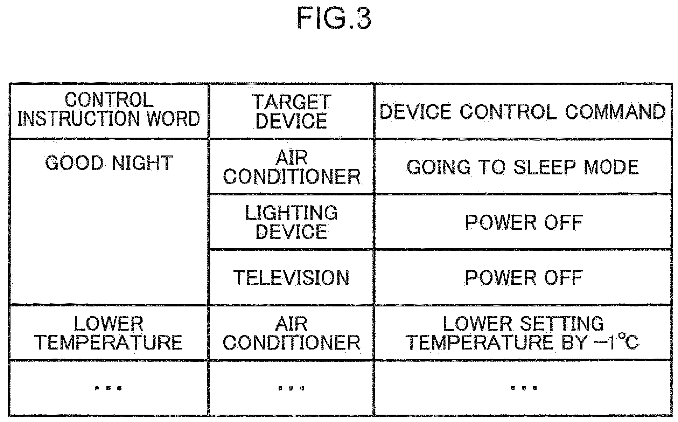

FIG. 3 is a diagram showing one example of the command table stored in the command table storage unit in the first embodiment of the present disclosure.

Examples of the control instruction word include "Good night", "Lower temperature" and "Good morning". As shown in FIG. 3, the control instruction word "Good night" is associated with the target device "air conditioner" and the device control command "going to sleep mode", associated with the target device "lighting device" and the device control command "power off", and associated with the target device "television" and the device control command "power off".

The command control unit 223 controls the output of the device control command based on the text data by controlling the necessity of operation determination to determine whether a predetermined operation has been performed by the user based on the sleep state. In the first embodiment, the predetermined operation is speaking the predetermined wake word by the user 103.

The command control unit 223 includes an operation determination necessity determination unit 224 (second determination unit), a wake word detection unit 225, and a control command generation unit 226.

The operation determination necessity determination unit 224 determines the necessity of operation determination based on the sleep state. That is, the operation determination necessity determination unit 224 determines whether the sleep state is the predetermined sleep state. Then, when it is determined that the sleep state is the predetermined sleep state, the operation determination necessity determination unit 224 determines that the operation determination is unnecessary, and when it is determined that the sleep state is not the predetermined sleep state, the operation determination necessity determination unit 224 determines that the operation determination is necessary. Here, the predetermined sleep state is, for example, any one of the lying in bed state, the pre-sleep awake state, the nocturnal awakening state, the nocturnal awakening sign state, the halfway leaving bed state, and the pre-rising awake state.

The operation determination necessity determination unit 224 determines which of the lying in bed state, the pre-sleep awake state, the nocturnal awakening state, the nocturnal awakening sign state, the halfway leaving bed state, and the pre-rising awake state is the sleep state of the user 103. Then, when it is determined that the sleep state is any one of the lying in bed state, the pre-sleep awake state, the nocturnal awakening state, the nocturnal awakening sign state, the halfway leaving bed state, and the pre-rising awake state, the operation determination necessity determination unit 224 determines that the operation determination using the wake word is unnecessary.

Note that the operation determination necessity determination unit 224 may determine whether the sleep state is the going to sleep state, and when it is determined that the sleep state is the going to sleep state, the operation determination necessity determination unit 224 may determine that the operation determination is unnecessary. The going to sleep state is a state from when the user lies in bed until when the user rises. For example, the going to sleep state may be a state from when the user enters bed until when the user goes out of bed for rising, or may be a state from when the user enters futon until when the user goes out of futon for rising. Examples of the going to sleep state include the lying in bed state, the pre-sleep awake state, the nocturnal awakening state, the nocturnal awakening sign state, the halfway leaving bed state, and the pre-rising awake state. Since this allows the device to be controlled without predetermined operation even in a stage of sleep preparation, it is possible to suppress the awakening degree of a person who is about to sleep from increasing. Even in the stage of preparation for rising from bed, it is possible to suppress the person's awakening degree from increasing before rising from bed completely.

The wake word storage unit 232 stores in advance the wake word for starting device control by voice. The wake word is, for example, "computer".

The wake word detection unit 225 detects the wake word stored in advance in the wake word storage unit 232 from the text data converted by the voice recognition unit 222. On detection of the wake word, the wake word detection unit 225 outputs a wake word detection signal to the control command generation unit 226.

Note that the predetermined operation in the first embodiment is the speaking of the predetermined wake word by the user 103, but the present disclosure is not particularly limited to this example. The predetermined operation may be either a predetermined gesture by the user 103 or predetermined line-of-sight movement by the user 103. In this case, the voice control system includes a camera that captures images of the user 103 in the bedroom 1. The camera transmits captured image data to the server 2. Then, the command control unit 223 includes a gesture detection unit that detects a predetermined gesture by the user 103 from the image data captured by the camera. The command control unit 223 includes a line-of-sight detection unit that detects predetermined line-of-sight movement by the user 103 from the image data captured by the camera. When it is determined that the sleep state is the predetermined sleep state, the operation determination necessity determination unit 224 determines that operation determination using a gesture or eye movement is unnecessary.

When it is determined by the operation determination necessity determination unit 224 that the operation determination is necessary, the control command generation unit 226 determines whether the predetermined operation has been performed by the user 103, and outputs the device control command when it is determined that the predetermined operation has been performed. That is, when it is determined by the operation determination necessity determination unit 224 that the operation determination is necessary, the control command generation unit 226 waits for the wake word detection signal output from the wake word detection unit 225. When the wake word detection signal is input from the wake word detection unit 225, the control command generation unit 226 refers to the command table stored in the command table storage unit 231, and generates the device control command from the text data that follows the wake word out of the acquired text data.

When it is determined by the operation determination necessity determination unit 224 that the operation determination is unnecessary, the operation determination necessity determination unit 224 does not determine whether the predetermined operation has been performed by the user 103, and outputs the device control command. That is, when it is determined by the operation determination necessity determination unit 224 that the operation determination is unnecessary, the operation determination necessity determination unit 224 refers to the command table stored in the command table storage unit 231 and generates the device control command from the text data.

For example, if the user 103 speaks "Good night", the control command generation unit 226 generates a device control command for operating the air conditioner 14 in the going to sleep mode, generates a device control command for turning off the lighting device 15, and generates a device control command for turning off the television.

When it is determined by the operation determination necessity determination unit 224 that the sleep state is the predetermined sleep state, the control command generation unit 226 generates the device control command based on the predetermined sleep state. When it is determined that the sleep state of the user 103 is the predetermined sleep state, the control command generation unit 226 generates the device control command according to the time length from the scheduled rising time of the user 103 to the current time. For example, in a case where the user 103 speaks "Turn on the light", if the time from the scheduled rising time of the user 103 to the current time is within 15 minutes, the control command generation unit 226 generates the device control command for gradually increasing illuminance of the lighting device 15 on the ceiling of the bedroom 1 by the scheduled rising time. For example, in a case where the user 103 speaks "Turn on the light", if the time from the scheduled rising time of the user 103 to the current time is longer than 15 minutes, the control command generation unit 226 generates the device control command for lighting the lighting device 15 at the feet at low illuminance or low color temperature.

Furthermore, when it is determined that the sleep state is the predetermined sleep state, the control command generation unit 226 generates only the predetermined device control command related to going to sleep of the user 103. That is, when it is determined that the sleep state is the lying in bed state, the control command generation unit 226 receives only the specific control instruction word and generates only the predetermined device control command associated with the specific control instruction word. The user 103 may watch television between lying in bed and going to sleep. In this case, there is a possibility that the voice output from the television is collected by the microphone 121. When the sleep state is the lying in bed state, since the device is controlled by voice without performing operation determination using the wake word, there is a possibility that the device may be erroneously operated by the voice output from the television. Therefore, when it is determined that the sleep state is the lying in bed state, the control command generation unit 226 receives only the specific control instruction word. The control command generation unit 226 receives, for example, only control instruction words related to going to sleep, such as "Good night".

Note that when it is determined that the sleep state is the lying in bed state, the control command generation unit 226 may receive only the specific control instruction word for a predetermined period. For example, when it is determined that the sleep state is the lying in bed state, the control command generation unit 226 may receive only the specific control instruction word for 30 minutes. Then, after the predetermined period elapses, the control command generation unit 226 may receive not only the specific control instruction word but also other control instruction words.

When it is determined that the sleep state is the predetermined sleep state, the control command generation unit 226 may detect the control instruction word without detecting the wake word until a predetermined period elapses. Then, when the predetermined period elapses after it is determined that the sleep state is the predetermined sleep state, the control command generation unit 226 may detect the control instruction word after detecting the wake word.

When it is determined that the sleep state is the lying in bed state, the control command generation unit 226 may detect the control instruction word without detecting the wake word until a predetermined period elapses. Then, when the predetermined period elapses after it is determined that the sleep state is the lying in bed state, the control command generation unit 226 may detect the control instruction word after detecting the wake word.

When it is determined that the sleep state is the pre-sleep awake state, until the sleep state changes from the pre-sleep awake state to the REM sleep state, the light sleep state, or the deep sleep state, the control command generation unit 226 may detect the control instruction word without detecting the wake word. Then, when the sleep state changes from the pre-sleep awake state to the REM sleep state, the light sleep state, or the deep sleep state, the control command generation unit 226 may detect the control instruction word after detecting the wake word.

When it is determined that the sleep state is the nocturnal awakening sign state, the control command generation unit 226 may detect the control instruction word without detecting the wake word until a predetermined period elapses. Then, when the predetermined period elapses after it is determined that the sleep state is the nocturnal awakening sign state, the control command generation unit 226 may detect the control instruction word after detecting the wake word.

When it is determined that the sleep state is the nocturnal awakening sign state, the control command generation unit 226 may detect the control instruction word without detecting the wake word until the sleep state becomes a sleep state different from the nocturnal awakening sign state. Then, when the sleep state becomes a sleep state different from the nocturnal awakening sign state, the control command generation unit 226 may detect the control instruction word after detecting the wake word.

When it is determined that the sleep state is the nocturnal awakening state, the control command generation unit 226 may detect the control instruction word without detecting the wake word until a predetermined period elapses. Then, when the predetermined period elapses after it is determined that the sleep state is the nocturnal awakening state, the control command generation unit 226 may detect the control instruction word after detecting the wake word.

When it is determined that the sleep state is the nocturnal awakening state, until the sleep state changes from the nocturnal awakening state to the REM sleep state, the light sleep state, or the deep sleep state, the control command generation unit 226 may detect the control instruction word without detecting the wake word. Then, when the sleep state changes from the nocturnal awakening state to the REM sleep state, the light sleep state, or the deep sleep state, the control command generation unit 226 may detect the control instruction word after detecting the wake word.

When it is determined that the sleep state is the halfway leaving bed state, the control command generation unit 226 may detect the control instruction word without detecting the wake word until a predetermined period elapses. Then, when the predetermined period elapses after it is determined that the sleep state is the halfway leaving bed state, the control command generation unit 226 may detect the control instruction word after detecting the wake word.

When it is determined that the sleep state is the halfway leaving bed state, the control command generation unit 226 may detect the control instruction word without detecting the wake word until the sleep state changes from the halfway leaving bed state to the lying in bed state.

When it is determined that the sleep state is the pre-rising awake state, the control command generation unit 226 may detect the control instruction word without detecting the wake word until the sleep state changes from the pre-rising awake state to the leaving bed state. Then, when the sleep state changes from the pre-rising awake state to the leaving bed state, the control command generation unit 226 may detect the control instruction word after detecting the wake word.

When it is determined that the sleep state is the pre-rising awake state, the control command generation unit 226 may detect the control instruction word without detecting the wake word from the time when the sleep state changes from the pre-rising awake state to the leaving bed state until a predetermined period elapses. Then, when the predetermined period elapses from the time when the sleep state changes from the pre-rising awake state to the leaving bed state, the control command generation unit 226 may detect the control instruction word after detecting the wake word.

The communication unit 21 transmits the device control command to the device associated with the device control command.

An information processing method by the server 2 configured as described above will be described below.

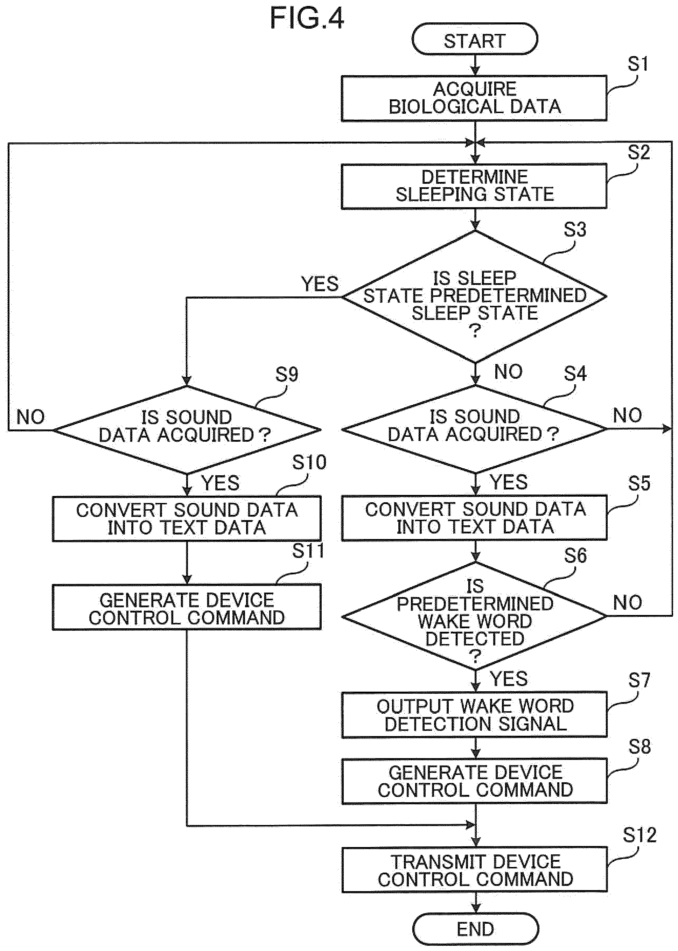

FIG. 4 is a flowchart showing one example of the information processing method by the server in the first embodiment of the present disclosure.

To begin with, in step S1, the communication unit 21 acquires the biological data of the user 103 from the biosensor 11. The biosensor 11 measures the heart rate, the respiratory rate, and the body movement amount of the user 103, and transmits the biological data including the measured heart rate, the respiratory rate, and the body movement amount to the server 2.

Next, in step S2, the sleep state determination unit 221 determines the sleep state of the user 103 based on the biological data from the biosensor 11. Here, based on the biological data, the sleep state determination unit 221 estimates which of the leaving bed state, the lying in bed state, the awake state, the pre-sleep awake state, the REM sleep state, the light sleep state, the deep sleep state, the nocturnal awakening state, the nocturnal awakening sign state, the halfway leaving bed state, and the pre-rising awake state is the sleep state of the user 103.

Next, in step S3, the operation determination necessity determination unit 224 determines whether the sleep state determined by the sleep state determination unit 221 is the predetermined sleep state. The predetermined sleep state is, for example, any one of the lying in bed state, the pre-sleep awake state, the nocturnal awakening state, the nocturnal awakening sign state, the halfway leaving bed state, and the pre-rising awake state. Here, when it is determined that the sleep state is not the predetermined sleep state (NO in step S3), in step S4, the voice recognition unit 222 determines whether the sound data is acquired by the communication unit 21. Here, when it is determined that the sound data is not acquired (NO in step S4), the process returns to step S2.

On the other hand, when it is determined that the sound data is acquired (YES in step S4), in step S5, the voice recognition unit 222 converts the acquired sound data into text data.

Next, in step S6, the wake word detection unit 225 determines whether the predetermined wake word is detected from the text data converted by the voice recognition unit 222. Here, when it is determined that the predetermined wake word is not detected (NO in step S6), the process returns to step S2.

On the other hand, when it is determined that the predetermined wake word is detected (YES in step S6), in step S7, the wake word detection unit 225 outputs the wake word detection signal to the control command generation unit 226.

Next, in step S8, with reference to the command table stored in the command table storage unit 231, the control command generation unit 226 detects the control instruction word from the text data following the wake word out of the acquired text data, and generates the device control command corresponding to the control instruction word. Note that when the control instruction word is not detected from the acquired text data, the process may return to step S2.

Note that in the first embodiment, after the wake word is detected from the text data, the control command generation unit 226 detects the control instruction word included in the text data and generates the device control command corresponding to the detected control instruction word, but the present disclosure is not particularly limited to this example. To begin with, the control command generation unit 226 may detect the control instruction word included in the text data. Then, after the control instruction word is detected, the wake word detection unit 225 may detect the wake word from the text data before the detected control instruction word. Then, when the wake word is detected, the control command generation unit 226 may generate the device control command corresponding to the control instruction word.

On the other hand, when it is determined in step S3 that the sleep state is the predetermined sleep state (YES in step S3), in step S9, the voice recognition unit 222 determines whether the sound data is acquired by the communication unit 21. Here, when it is determined that the sound data is not acquired (NO in step S9), the process returns to step S2.

On the other hand, when it is determined that the sound data is acquired (YES in step S9), in step S10, the voice recognition unit 222 converts the acquired sound data into text data.

Next, in step S11, with reference to the command table stored in the command table storage unit 231, the control command generation unit 226 detects the control instruction word from the acquired text data, and generates the device control command corresponding to the control instruction word. Note that when the control instruction word is not detected from the acquired text data, the process may return to step S2.

Next, in step S12, the communication unit 21 transmits the device control command to the device associated with the device control command. The device is operated at the received device control command.

In this way, in the first embodiment, the user 103 can control the device, for example, by voice without predetermined operations such as speaking the wake word, making it possible to suppress the increase in the awakening degree of the user 103 while maintaining the convenience of device control during the sleeping period. The necessity of predetermined operation is controlled according to the sleep state of the user 103, making it possible to suppress erroneous control of the device without predetermined operations, which leads to an increase in the convenience of controlling the device by the user's voice.

Note that in the first embodiment, when it is determined that the sleep state is the predetermined sleep state, the control command generation unit 226 generates the device control command without detecting the predetermined wake word, but the present disclosure is not particularly limited to this example. When it is determined that the sleep state is the predetermined sleep state, the control command generation unit 226 may generate the device control command without detecting the predetermined wake word until the predetermined period elapses. Then, when the predetermined period elapses after it is determined that the sleep state is the predetermined sleep state, the control command generation unit 226 may generate the device control command after detecting the predetermined wake word.

### (Second Embodiment)

Subsequently, a voice control system in a second embodiment of the present disclosure will be described. In the second embodiment, the necessity of operation determination is determined in consideration of an environmental state in space in which a user goes to sleep.

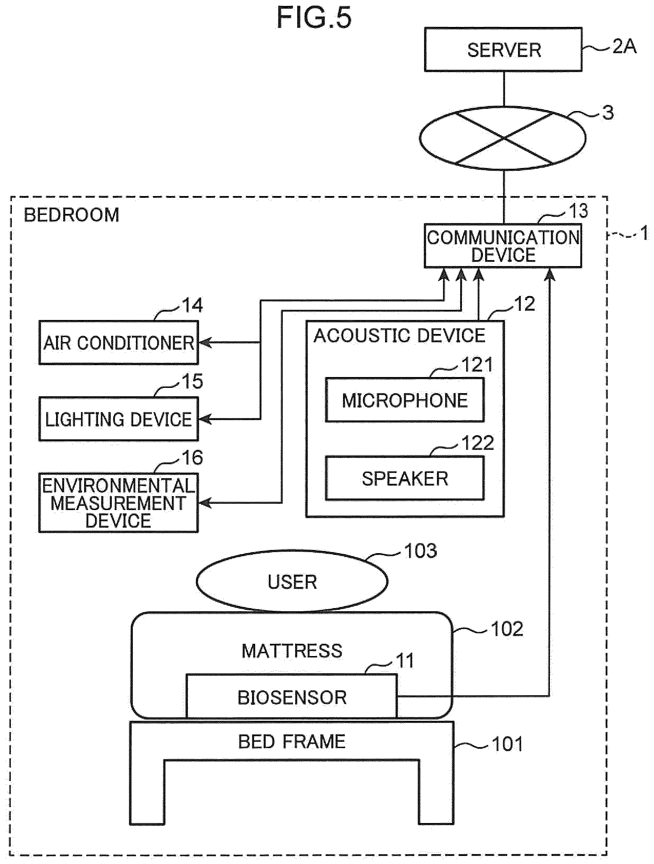

FIG. 5 is a diagram showing one example of a configuration of the voice control system in the second embodiment of the present disclosure.

In FIG. 5, the voice control system includes a server 2A, a biosensor 11, an acoustic device 12, a communication device 13, an air conditioner 14, a lighting device 15, and an environmental measurement device 16. The biosensor 11, the acoustic device 12, the communication device 13, the air conditioner 14, the lighting device 15, and the environmental measurement device 16 are disposed in a bedroom 1. Note that in the second embodiment, components identical to components of the first embodiment are denoted with identical reference signs, and descriptions thereof will be omitted.

The environmental measurement device 16 is preferably disposed, for example, near a user 103 such as near a pillow. The environmental measurement device 16 measures an environmental state in space in which the user 103 is present. The environmental measurement device 16 transmits environmental information indicating the environmental state in the space in which the user 103 is present to the server 2A. The environmental measurement device 16 is, for example, a temperature sensor. The environmental measurement device 16 measures the temperature in the bedroom 1 and transmits temperature information as the environmental information to the server 2A. The environmental measurement device 16 transmits the environmental information to the server 2A via the communication device 13. The environmental measurement device 16 is communicably connected to the server 2A via a network 3.

The environmental measurement device 16 may be a terminal device owned by the user 103. The terminal device is, for example, a smartphone, a tablet computer, or a wearable terminal. Any one of the biosensor 11, the acoustic device 12, the air conditioner 14, and the lighting device 15 may include the environmental measurement device 16.

Note that the environmental measurement device 16 measures the temperature in the bedroom 1 as the environmental state, but the present disclosure is not particularly limited to this example. The environmental measurement device 16 may measure other environmental states such as humidity or pollen amount in the bedroom 1.

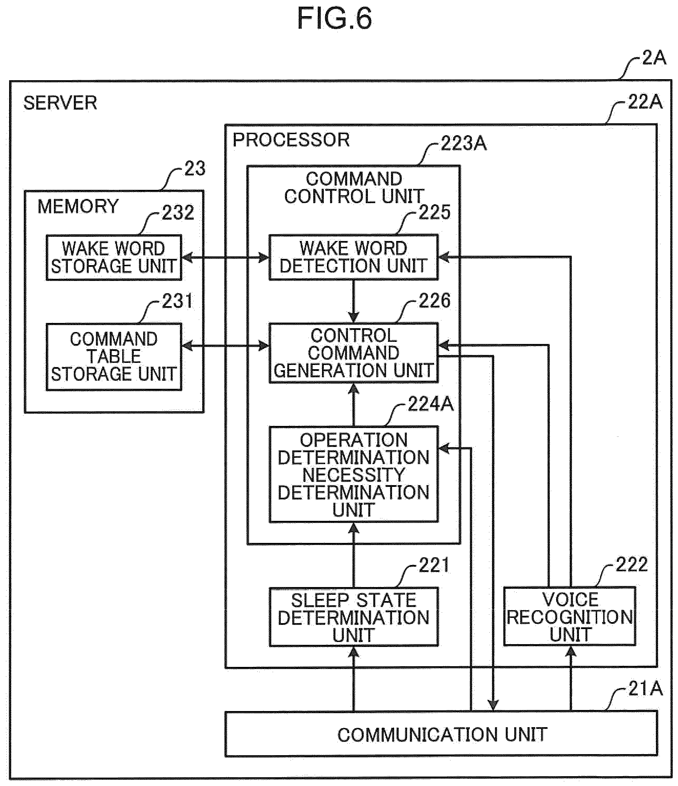

FIG. 6 is a diagram showing a configuration of the server in the second embodiment of the present disclosure.

The server 2A shown in FIG. 6 includes a communication unit 21A, a processor 22A, and a memory 23.

The communication unit 21A acquires biological data from the biosensor 11. The communication unit 21A receives the biological data transmitted by the biosensor 11. The communication unit 21A acquires sound data from the microphone 121. The communication unit 21A receives the sound data transmitted by the acoustic device 12. Furthermore, the communication unit 21A acquires the environmental information from the environmental measurement device 16. The communication unit 21A receives the environmental information transmitted by the environmental measurement device 16.

The processor 22A includes a sleep state determination unit 221, a voice recognition unit 222, and a command control unit 223A. The command control unit 223A includes an operation determination necessity determination unit 224A, a wake word detection unit 225, and a control command generation unit 226.

The operation determination necessity determination unit 224A determines the necessity of operation determination for determining whether a predetermined operation has been performed by the user 103 based on a sleep state and the environmental information.

In the first embodiment, when it is determined by the operation determination necessity determination unit 224 that the sleep state of the user 103 is a nocturnal awakening state, a control instruction word is detected without detection of a predetermined wake word.

Here, if the temperature in the bedroom 1 is suitable for comfortable sleep, the user 103 who awakes halfway during the sleeping period from lying in bed to rising is likely to fall asleep immediately. On the other hand, if the temperature in the bedroom 1 is not suitable for comfortable sleep, the user 103 who awakes halfway during the sleeping period has the possibility of awakening due to the uncomfortable temperature in the bedroom 1 and is likely to fail to fall asleep for some time.

Therefore, when it is determined that the sleep state of the user 103 is the nocturnal awakening state, the operation determination necessity determination unit 224A of the second embodiment determines whether the temperature in the bedroom 1 is lower than a predetermined value. When it is determined by the operation determination necessity determination unit 224A that the sleep state is the nocturnal awakening state and that the temperature in the bedroom 1 is lower than the predetermined value, the control command generation unit 226 detects the control instruction word without detecting the predetermined wake word. On the other hand, when it is determined by the operation determination necessity determination unit 224A that the sleep state is the nocturnal awakening state but that the temperature in the bedroom 1 is equal to or higher than the predetermined value, the control command generation unit 226 detects the control instruction word after detecting the predetermined wake word. Note that the predetermined value is a temperature at which comfortable sleep can be obtained. The predetermined value in the summer season is, for example, 25°C. The predetermined value in the winter season is, for example, 18°C.

In this way, in the second embodiment, it is possible to determine the necessity of operation determination by considering not only the sleep state but also whether the environmental state in the space is suitable for sleeping.

Note that in each of the embodiments described above, each component may include dedicated hardware or may be implemented by executing a software program suitable for the component. Each component may be implemented by a program execution unit such as a CPU or a processor reading and executing a software program recorded on a recording medium, such as a hard disk or a semiconductor memory.

Part or all of functions of the device according to the embodiments of the present disclosure are typically implemented as a large scale integration (LSI), which is an integrated circuit, These functions may be formed as separate chips, or some or all of the functions may be included in one chip. The circuit integration is not limited to LSI, and may be implemented using a dedicated circuit or a general-purpose processor. A field programmable gate array (FPGA) that is programmable after manufacturing of an LSI or a reconfigurable processor in which connections and settings of circuit cells within the LSI are reconfigurable may be used.

Part or all of functions of the device according to the embodiments of the present disclosure may be implemented by a processor such as a CPU executing a program.

All numerical values used above are merely illustrative to be used to specifically describe the present disclosure, and thus the present disclosure is not limited to the illustrative numerical values.

Order in which steps shown in the flowcharts are executed is merely illustrative to be used to specifically describe the present disclosure, and thus steps may be executed in order other than the above order as long as similar effects are obtained. Some of the steps may be executed simultaneously (in parallel) with other steps.

### Industrial Applicability

The technique according to the present disclosure, which can suppress the increase in the user's awakening degree while maintaining the convenience of device control during the sleeping period, is useful for the technique of controlling devices by the user's voice.

## Claims

1. An information processing method comprising, by a computer:
acquiring biological data from a biosensor that senses a biological state of a user;
acquiring sound data from a microphone that senses sound in space in which the user is present;
determining a sleep state of the user based on the biological data;
converting the sound data into text data; and
controlling output of a device control command based on the text data by controlling necessity of operation determination to determine whether a predetermined operation has been performed by the user based on the sleep state.

2. The information processing method according to claim 1, wherein the control of the output of the device control command includes:
determining the necessity of the operation determination based on the sleep state;
when it is determined that the operation determination is necessary,
determining whether the predetermined operation has been performed by the user, and
outputting the device control command when it is determined that the predetermined operation has been performed; and
when it is determined that the operation determination is unnecessary, outputting the device control command.

3. The information processing method according to claim 2, further comprising acquiring environmental information indicating an environmental state in the space,
wherein the control of the output of the device control command includes determining the necessity of the operation determination based on the sleep state and the environmental information.

4. The information processing method according to claim 2 or 3, wherein the control of the output of the device control command includes transmitting the device control command to a device associated with the device control command.

5. The information processing method according to any one of claims 2 to 4, wherein
the determination of the necessity of the operation determination includes: determining whether the sleep state is a lying in bed state in which the user lies in bed; and determining that the operation determination is unnecessary when it is determined that the sleep state is the lying in bed state.

6. The information processing method according to any one of claims 2 to 5, wherein
the determination of the necessity of the operation determination includes: determining whether the sleep state is a nocturnal awakening state in which the user awakes during a sleeping period from lying in bed to rising; and determining that the operation determination is unnecessary when it is determined that the sleep state is the nocturnal awakening state.

7. The information processing method according to any one of claims 2 to 6, wherein
the determination of the necessity of the operation determination includes: determining whether the sleep state is a nocturnal awakening sign state indicating a sign of shifting to the nocturnal awakening state in which the user awakes during the sleeping period from lying in bed to rising; and determining that the operation determination is unnecessary when it is determined that the sleep state is the nocturnal awakening sign state.

8. The information processing method according to any one of claims 2 to 7, wherein
the determination of the necessity of the operation determination includes: determining whether the sleep state is a first awake state from when the user lies in bed to falling asleep; and determining that the operation determination is unnecessary when it is determined that the sleep state is the first awake state.

9. The information processing method according to any one of claims 2 to 8, wherein
the determination of the necessity of the operation determination includes: determining whether the sleep state is a second awake state during a period from a scheduled rising time of the user to a predetermined time; and determining that the operation determination is unnecessary when it is determined that the sleep state is the second awake state.

10. The information processing method according to any one of claims 2 to 4, wherein
the determination of the necessity of the operation determination includes: determining whether the sleep state is a state from when the user lies in bed to rising; and determining that the operation determination is unnecessary when it is determined that the sleep state is the state from when the user lies in bed to rising.

11. The information processing method according to any one of claims 5 to 10, wherein the control of the output of the device control command includes generating the device control command based on the predetermined sleep state when it is determined that the sleep state is the predetermined sleep state.

12. The information processing method according to claim 11, wherein the generation of the device control command includes generating the device control command according to a time length from the scheduled rising time of the user to current time when it is determined that the sleep state is the predetermined sleep state.

13. The information processing method according to any one of claims 5 to 10, wherein the control of the output of the device control command includes generating only the predetermined device control command related to going to sleep of the user when it is determined that the sleep state is the predetermined sleep state.

14. The information processing method according to any one of claims 1 to 13, wherein the predetermined operation includes any of speaking a predetermined wake word by the user, a predetermined gesture by the user, and predetermined line-of-sight movement by the user.

15. An information processing system comprising:
a biosensor configured to sense a biological state of a user;
a microphone configured to sense a sound in space in which the user is present; and
an information processing device,
wherein the information processing device includes:
a biological data acquisition unit configured to acquire biological data from the biosensor;
a sound data acquisition unit configured to acquire sound data from the microphone;
a sleep state determination unit configured to determine a sleep state of the user based on the biological data;
a conversion unit configured to convert the sound data into text data; and
an output control unit configured to control output of a device control command based on the text data by controlling necessity of operation determination to determine whether a predetermined operation has been performed by the user based on the sleep state.

16. An information processing program for causing a computer to perform functions of:
acquiring biological data from a biosensor that senses a biological state of a user;
acquiring sound data from a microphone that senses sound in space in which the user is present;
determining a sleep state of the user based on the biological data;
converting the sound data into text data; and
controlling output of a device control command based on the text data by controlling necessity of operation determination to determine whether a predetermined operation has been performed by the user based on the sleep state.
